# EUROPÄISCHE PATENTANMELDUNG

(11) **EP 4 159 330 A1**
(43) Veröffentlichungstag der Anmeldung: **05.04.2023**
(21) Anmeldenummer: 22000095.4
(22) Anmeldetag: 18.02.2021
(51) Int. Cl.: B08B 1/00, B08B 1/02, B08B 7/00, B65G 45/22, B65G 45/10, A61L 2/10

(54) **REINIGUNGSANLAGE FÜR EIN VORLAUFBAND EINES KASSENTISCHES**

(30) Priorität: 29.04.2020 DE 102020111609
(62) Teilanmeldung aus: 21000051.9
(71) Anmelder: HARTING Systems GmbH, 32339 Espelkamp (DE)
(72) Erfinder: Pleye, Christof, 49324 Melle (DE)

(57) **Zusammenfassung**

Die Erfindung betrifft eine Reinigungseinheit (5, 5') für ein Warenvorlaufband (2) eines Kassentisches (1), wobei die Reinigungseinheit (5) Mittel zur UV-Bestrahlung des Warenvorlaufbandes (2) aufweist.

## Beschreibung

Die Erfindung geht aus von einer Reinigungseinheit für ein Warenvorlaufband eines Kassentisches nach der Gattung des unabhängigen Anspruchs 1. Die Erfindung bezieht sich weiterhin auf einen Kassentisch, der eine solche Reinigungseinheit aufweist, nach Anspruch 8.

Derartige Reinigungseinheiten werden zur Säuberung bzw. Desinfektion von Warenvorlaufbändern von Kassentischen, welche beispielsweise in Supermärkten angeordnet sind, eingesetzt.

### Stand der Technik

Warenvorlaufbänder von Kassentischen sind einer ständigen Verschmutzung, beispielsweise durch undichte Lebensmittelverpackungen ausgesetzt. Außerdem werden die Warenvorlaufbänder permanent von einer Vielzahl verschiedener Personen berührt, wodurch die Oberfläche des Warenvorlaufbandes auch Krankheitserreger aufweist. Die Warenvorlaufbänder müssen regelmäßig vom Kassenpersonal händisch gereinigt werden. Die Kassentische können während der Reinigung nicht eingesetzt werden, was zu längeren Warteschlagen und letztendlich auch zu Umsatzeinbußen führen kann.

Die US 2006/0219524 A1 zeigt eine Reinigungsvorrichtung für ein handelsübliches Förderband, das auf einem Fördertisch montiert ist. Die Reinigungsvorrichtung umfasst eine Wischvorrichtung. Die Wischvorrichtung ist so konstruiert, dass sie ein Wegwerfhandtuch, wie etwa ein Papierhandtuch, auf das Förderband aufbringt, um ein zuvor aufgespritztes Lösungsmittel einschließlich aller Verunreinigungen zu entfernen, während das Förderband in Bewegung ist.

Die US 2011/067978 A1 zeigt eine Vorrichtung zum Reinigen eines Förderbandes mit einem Behälter. Der Behälter umfasst eine Kammer, die zum Speichern eines Reinigungsfluids vorgesehen ist.

Die US 9,096,392 B1 zeigt eine Förderband-Dampfreinigungsvorrichtung zum Reinigen eines Endlosschleifen-Förderbandes, insbesondere umfassend eine Dampfsprüheinheit und eine Wischeinheit.

Die US 2010/243410 A1 zeigt eine Förderband-Reinigungs- und -Desinfektionseinheit, umfassend eine hohle poröse Walze, die auf der verdeckten Seite oder Rücklaufschleifenseite des Förderbands außerhalb der Sichtweite der oberen oder Förderfläche des Bands angeordnet ist.

Die US 2005/109580 A1 zeigt eine Förderband-Reinigungsvorrichtung umfassend eine Schmutzentfernungsstufe, eine Waschstufe, eine Spülstufe und eine Wasserentfernungsstufe, die alle in einer Schublade mit mehreren Kammern positioniert sind, wobei jede der vier Stufen mit einem isolierten Behälter innerhalb dieser Schublade versehen ist.

Die US 2012/211645 A1 zeigt ein Sterilisationssystem zum Sterilisieren eines Endlosschleifen-Förderbandes. Das Sterilisationssystem umfasst ein Gehäuse in welchem eine UV-Lichtquelle positioniert ist.

Insbesondere in Zeiten von weltweiten Pandemien ist die Erfordernis für besondere Hygienemaßnahmen stark angestiegen.

### Aufgabenstellung

Die Aufgabe der Erfindung besteht darin eine zuverlässige und ein Gesundheitsrisiko verringernde Reinigungseinheit für ein Warenvorlaufband eines Kassentisches vorzuschlagen. Die Aufgabe wird durch den Gegenstand der unabhängigen Ansprüche gelöst.

Vorteilhafte Ausgestaltungen der Erfindung sind in den Unteransprüchen und der folgenden Beschreibung angegeben.

Die erfindungsgemäße Reinigungseinheit ist für die Reinigung eines Warenvorlaufbandes eines Kassentisches vorgesehen. Mit Reinigung ist nicht nur das Entfernen von Schmutz gemeint. Mit einer erfindungsgemäßen Reinigung ist insbesondere auch das Verringern von Bakterien- und Viruskonzentration auf der Oberfläche des Warenvorlaufbandes gemeint.

Die Reinigung ist mit Mitteln zur Nassreinigung und/oder mit Mittel zur UV-Bestrahlung des Warenvorlaufbandes ausgestattet. Bei der Nassreinigung werden Schmutzpartikel physikalisch von der Oberfläche des Warenvorlaufbandes entfernt. Je nach verwendeter Reinigungsflüssigkeit werden hierbei auch Bakterien und/oder Viren reduziert oder entfernt bzw. unschädlich gemacht.

Eine permanente physikalische Reinigung ist jedoch nicht immer sinnvoll, da sie den Verschleiß des Warenvorlaufbandes erhöhen kann, weil beispielsweise das Gummimaterial von der Reinigungsflüssigkeit schneller spröde wird. Außerdem sind hochwertige Reinigungsmittel sehr hochpreisig, was die Kosten für ein Nassreinigungsverfahren deutlich erhöht.

Die Bakterium- und Virenbelastung der Oberfläche des Warenvorlaufbandes kann effektiv mit geeigneter UV-Strahlung reduziert werden. Daher kann die erfindungsgemäße Reinigungseinheit mit einer entsprechenden Leuchteinheit, vorzugsweise einer UV-Strahlung aussendenden LED, ausgestattet sein.

In einer besonders bevorzugten Variante der Erfindung verfügt die Reinigungseinheit über Mittel zur Nassreinigung und gleichzeitig über Mittel zur UV-Bestrahlung der Oberfläche des Warenvorlaufbandes. Dadurch kann eine besonders zuverlässige Reinigung des Warenvorlaufbandes gewährleistet werden.

Besonders bevorzugt werden die verschiedenen Reinigungsmittel sequentiell eingesetzt. Eine Nassreinigung kann sich mit einer UV-Bestrahlung abwechseln.

Es kann aber auch vorteilhaft sein, wenn die UV-Bestrahlung permanent durchgeführt wird und die Nassreinigung lediglich zwischendurch durchgeführt wird, beispielsweise dann, wenn das Warenvorlaufband einen gewissen Verschmutzungsgrad erreicht hat. Zur Detektion des Verschmutzungsgrades kann bei der Reinigungseinheit ein geeigneter Sensor vorgesehen sein.

Vorzugsweise weist die Reinigungseinheit eine Steuereinheit auf, die anhand der Sensordaten ein entsprechendes Reinigungsprogramm selbständig ermittelt. Dadurch ist der Oberfläche des Warenvorlaufbandes immer ausreichend sauber und desinfiziert ohne zu stark beansprucht zu werden.

Die Reinigungseinheit weist vorzugsweise einen Tank, in welchen ein Reinigungsmittel füllbar ist oder welcher bereits mit einem Reinigungsmittel gefüllt ist, auf. Als Reinigungsmittel kann ein in Wasser gelöstes Mittel dienen. Es kann sich dabei um ein viskoses Gel handeln oder schlichtweg um bereits eine vorgemischte Reinigungsflüssigkeit. Der Reinigungstank kann mit einem flüssigen Reinigungsmittel befüllt werden. Das Reinigungsmittel kann an die jeweiligen Reinigungsbedürfnisse angepasst sein. Beispielsweise kann eine wässrige Seifenlösung eine klebrig verschmutzte Oberfläche gut reinigen. Durch Zugabe eines antibakteriellen und/oder viruziden Reinigungsmittels kann die Oberfläche von gesundheitsgefährdenden Erregern befreit oder deren Anzahl bzw. Konzentration zumindest stark verringert werden.

Die Reinigungseinheit weist ein Reinigungstuch, welches flächig mit dem Warenvorlaufband in Berührkontakt bringbar ist, auf. Das Reinigungstuch kann für den jeweiligen Einsatzzweck ausgestaltet sein. Soll lediglich eine mechanische Reinigung erfolgen, kann das Reinigungstuch waschbar und damit widerverwertbar ausgestaltet sein. Soll bei der Reinigung auch eine Reduzierung von Krankheitserregern erreicht werden, bietet sich ein Einwegreinigungstuch an.

Vorzugsweise weist die Reinigungseinheit ein Klettband auf. Durch das Klettband kann das Reinigungstuch einfach an der Reinigungseinheit befestigt und schnell ausgetauscht werden.

Die Reinigungseinheit weist ein Tropfsystem, welches das Reinigungstuch zumindest bei einem umlaufenden Warenvorlaufband mit Reinigungsmittel durchfeuchtet, auf. Die Durchfeuchtung kann kontinuierlich oder in einem vorbestimmten Intervall erfolgen. Durch das Tropfsystem kann beispielsweise die Menge (Liter) an Reinigungsflüssigkeit pro Zeiteinheit (Sekunden) eingestellt werden, mit der das Reinigungstuch durchtränkt wird. Dadurch kann die Reinigungsleistung auf den jeweiligen Verschmutzungsgrad abgestimmt werden. Zu Stoßzeiten wird beispielsweise entsprechend mehr Reinigungsflüssigkeit pro Zeiteinheit (Milliliter/Sekunde) verwendet.

Vorzugsweise ist das Tropfsystem derart eingerichtet, dass sie durch ein Stoppen des Warenvorlaufbandes deaktivierbar ist. Das bedeutet, dass das Reinigungstuch nur bei laufenden Warenvorlaufband mit Reinigungsflüssigkeit durchtränkt wird. Das Tropfsystem kann' beispielsweise einen trichterförmigen Auslass aufweisen, der durch eine passende Kugel verschlossen ist. Durch den Druck der Reinigungsflüssigkeit wird die Kugel in die Auslassöffnung eingedrückt (deaktiviertes Tropfsystem). Durch die Bewegung des Warenvorlaufbandes wird die Kugel zurückgedrückt wodurch eine gewisse Menge an Reinigungsflüssigkeit entweichen kann (aktiviertes Tropfsystem).

Vorzugsweise weist die Reinigungseinheit zumindest ein Befestigungsmittel auf, mit welchem sie reversibel auf oder am Warenvorlaufband befestigbar ist. Bei diesem Befestigungsmittel kann es sich um zumindest einen Magneten und/oder zumindest einen Haken und/oder zumindest eine Schraube handeln.

Das Warenvorlaufband ist in der Regel in einem metallischen Gehäuse eingebettet, welches im Auflagebereich einen umlaufenden Rand ausbildet. Die Reinigungseinheit kann einfach auf das Warenvorlaufband aufgesetzt werden. Über eine Magnetfixierung kann die Reinigungseinheit an den metallischen Rand des Gehäuses des Warenvorlaufbandes reversibel fixiert werden. Alternativ kann ein Rückhalteelement am Beginn des Zulaufbandes angebracht werden. Als Rückhalteelement kann hierbei zumindest ein Haken verstanden werden, welcher an das Gehäuse des Warenvorlaufbands eingehakt wird. Zu Wartungszwecken kann die Reinigungseinheit einfach wieder abgenommen werden, ohne dass das Warenvorlaufband zwangsläufig angehalten werden muss. Eine Wartung der Reinigungseinheit im laufenden Kassenbetrieb ist demnach möglich.

In einer bevorzugten Variante der Erfindung ist der Tank dezentral angeordnet. Das bedeutet, dass der Tank nicht direkt mit den anderen Komponenten der Reinigungseinheit verbunden ist. Der Tank kann dann in einem ungenutzten Bauraum im Kassenbereich, beispielsweise unterhalb der Regalböden für Impulswaren, untergebracht sein. Über eine elektrische Pumpe erfolgt die Förderung des Reinigungsmittels und damit die Benetzung des Reinigungstuches. Die Abgabe des Reinigungsmittels kann separat gesteuert werden. Beispielsweise kann die Steuerung über das Kassenpersonal erfolgen, die die Zuführung des Reinigungsmittels bei Bedarf aktiviert. Dadurch kann die Reinigungseinheit sehr effizient eingesetzt werden.

### Ausführungsbeispiel

Ein Ausführungsbeispiel der Erfindung ist in den Zeichnungen dargestellt und wird im Folgenden näher erläutert. Es zeigen:
- Fig. 1: ein perspektivischer Ausschnitt eines Kassentisches mit einer Reinigungseinheit,
- Fig. 2: eine schematische Darstellung der Reinigungseinheit,
- Fig. 3: einen Ausschnitt eines Kassentisches mit einer integrierten Reinigungseinheit und
- Fig. 4: eine schematische Seitenansicht einer weiteren Ausführungsform einer Reinigungseinheit.

Die Figuren enthalten teilweise vereinfachte, schematische Darstellungen. Zum Teil werden für gleiche, aber gegebenenfalls nicht identische Elemente identische Bezugszeichen verwendet. Verschiedene Ansichten gleicher Elemente könnten unterschiedlich skaliert sein.

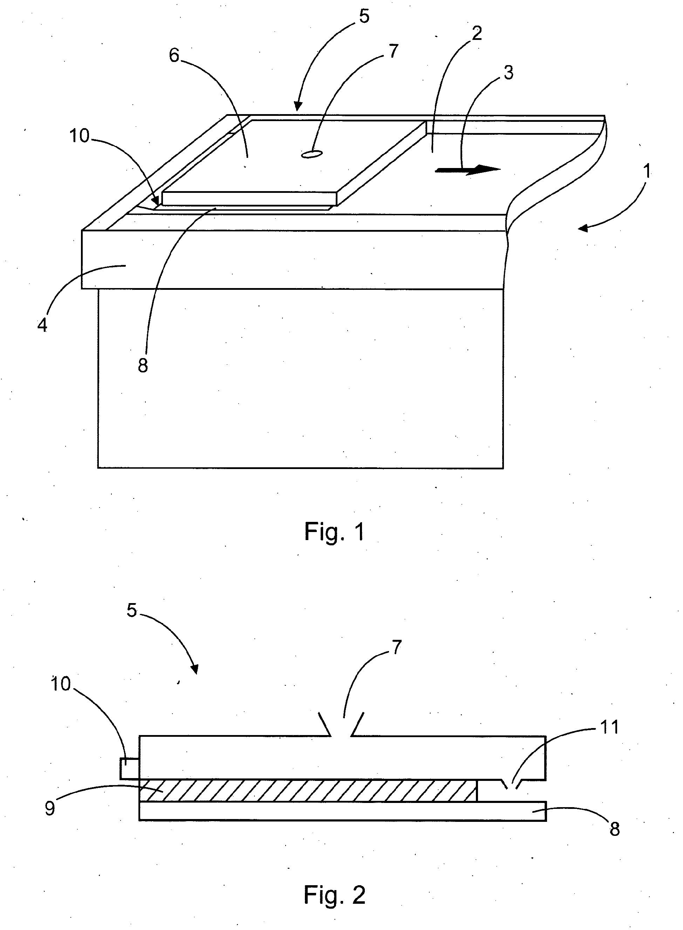

In Figur 1 ist aus darstellerischen Gründen lediglich ein Ausschnitt eines Kassentisches 1 dargestellt. Der Kassentisch 1 weist ein Warenvorlaufband 2 auf, welches die darauf befindliche Ware (nicht gezeigt) in Richtung des Pfeils 3 zu einer Kassenkraft bzw. Kassierperson (nicht gezeigt) transportiert.

Das Warenvorlaufband 2 wird über Walzen (nicht gezeigt) angetrieben.
Das gesamte System, bestehend aus dem umlaufenden Warenvorlaufband 2, den besagten Walzen und einer Antriebseinheit, sind in einem Gehäuse 4 untergebracht. Das Gehäuse 4 besteht in der Regel aus einem metallischen Material.

Auf das Warenvorlaufband 2 wird eine erfindungsgemäße Reinigungseinheit 5 einfach aufgesetzt. Die Reinigungseinheit 5 weist einen Tank 6 auf. Der Tank 6 weist an seiner Oberseite eine Einfüllöffnung 7 auf, durch welche ein Reinigungsmittel eingefüllt werden kann. Je nach Bedarf können verschiedene Reinigungsmittel für unterschiedliche Verschmutzungssituationen gewählt werden. Die Reinigungsmittel können auch in unterschiedlichen Lösungskonzentrationen genutzt werden, so dass eine Umweltbelastung reduziert werden kann, wenn ökologisch dosierte Lösungen verwendetet werden.

Die Reinigungseinheit 5 weist ein Reinigungstuch 8 auf. Das Reinigungstuch 8 ist unterhalb des Tanks 6 über ein Klettband 9 an selbigen befestigt. Das Reinigungstuch 8 liegt flächig auf dem Warenvorlaufband 2 auf.

Die Reinigungseinheit 5 verfügt über ein Tropfsystem 11, welches das Reinigungstuch 8 mit Reinigungsmittel durchfeuchtet. Diese Durchfeuchtung kann kontinuierlich erfolgen, indem stetig eine gewisse Reinigungsflüssigkeitsmenge pro Zeiteinheit in das Reinigungstuch 8 abgegeben wird und sich dieses mit der Reinigungsflüssigkeit gleichmäßig vollsaugt. Ein solche rein mechanische Lösung kann kostengünstig produziert werden und ist außerdem sehr wartungsarm.

In einer alternativen Variante kann das Tropfsystem 11 derart ausgestaltet sein, dass es nur bei einem laufenden Warenvorlaufband 2 Reinigungsmittel bzw. Reinigungsflüssigkeit in das Reinigungstuch 8 abgibt. Eine solche Steuerung kann über eine integrierte Steuereinheit (nicht gezeigt) elektromechanisch erfolgen.

Die Reinigungseinheit 5 weist einen Magneten 10 auf, mit welchem die Reinigungseinheit 5 am Gehäuse 4 des Warenvorlaufbandes 2 befestigt ist. Die Reinigungseinheit 5 kann dadurch an mehreren Kassentischen eingesetzt werden.

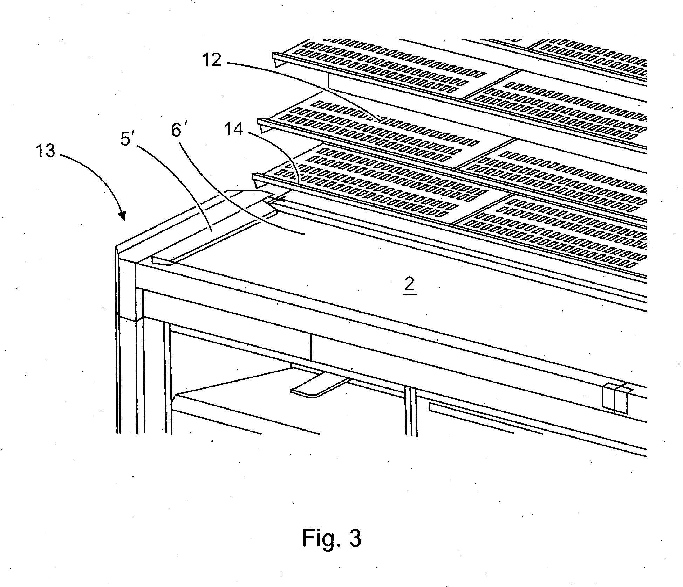

In Figur 3 ist ein perspektivischer Ausschnitt eines Kassentisches 13 zu sehen, der eine integrierte Reinigungseinheit 5' aufweist. In dem hier gezeigten Ausführungsbeispiel ist der Tank 6' der Reinigungseinheit 5' nicht direkt mit den restlichen Komponenten verbunden, sondern unterhalb des Regalsystems 12 des Kassentisches 13 untergebracht. Um den Tank 6' sehen zu können wird der Kassentisch 13 in Figur 3 teilweise transparent dargestellt. Über eine elektrische Pumpe 14 wird Reinigungsmittel aus dem Tank 6' zum Reinigungstuch (in Figur 3 nicht zu sehen) gefördert. Da der Tank 6' der Reinigungseinheit 5' in diesem Ausführungsbeispiel in einem ansonsten ungenutzten Bauraum untergebracht ist, kann der auf dem Warenvorlaufband 2 befindliche Teil der Reinigungseinheit 5' klein gebaut werden, so dass die Auflagefläche des Warenvorlaufbandes 2 hierdurch kaum verkleinert wird.

In Figur 3 ist eine besonders bevorzugte Ausführungsform einer Reinigungseinheit 5' für ein Warenvorlaufband 2 gezeigt. Die Reinigungseinheit 5' verfügt über Mittel zur Nassreinigung, also zur Reinigung des Warenvorlaufbandes 2 mithilfe eines Reinigungsmittels bzw. einer Reinigungsflüssigkeit.

Insbesondere zur Nassreinigung verfügt dieses zweite Ausführungsbeispiel über einen Sprühkopf 15, der innerhalb des Gehäuses 4' angeordnet ist. Durch den Sprühkopf 15 wird ein feiner Nebel 21 von geeignetem Reinigungsmittel auf der Oberfläche des Warenvorlaufbandes 2 breitflächig, aber lokal begrenzt verteilt.

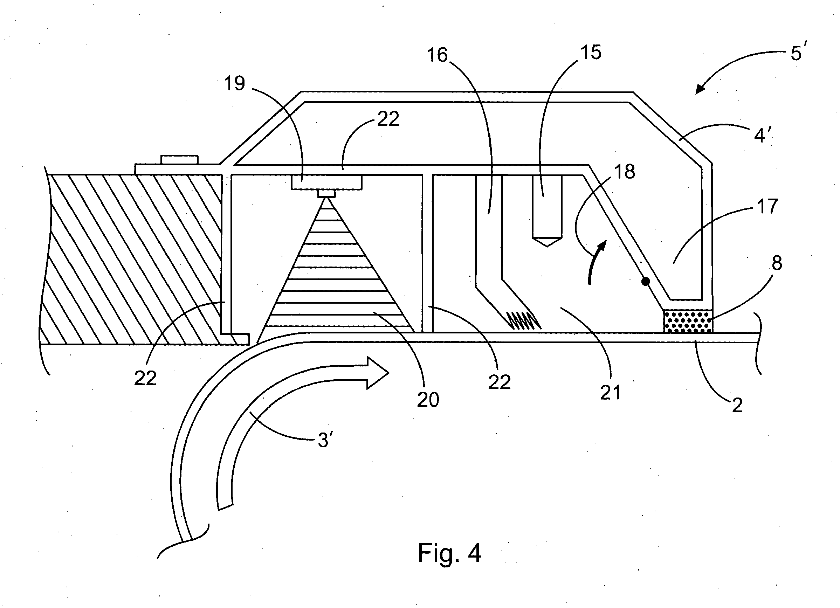

Grobe Verschmutzungen der Warenvorlaufbandoberfläche werden mit einer Bürste gelöst. Die gelösten Schmutzpartikel werden von einem Reinigungstuch 8 bzw. Reinigungsschwamm aufgefangen. Anstelle eines Reinigungsschwamms kann eine Reinigungsbürste 17 vorgesehen sein, die in Figur 4 lediglich gestrichelt angedeutet ist. Die Drehrichtung der Reinigungsbürste 17 wird durch den Pfeil 18 angedeutet.

Insbesondere zur UV-Bestrahlung verfügt dieses zweite Ausführungsbeispiel der Reinigungseinheit 5' über eine Leuchteinheit 19, die UV-Strahlung 20 bzw. elektromagnetische Wellen mit UV-Wellenlängenbereich aussendet. Hierbei handelt es sich bevorzugt um eine UV-LED. Die Leuchteinheit 19 ist innerhalb des Gehäuses 4' der Reinigungseinheit 5' angeordnet und derart ausgerichtet, dass die UV-Strahlung 20 die Oberfläche des Warenvorlaufbandes 2 breitflächig trifft.

Innerhalb des Gehäuses 4' sind zwei Abschirmwände 22 parallel zueinander angeordnet. Oberhalb werden die beiden Abschirmwände durch eine weitere Abschirmwand 22 verbunden, so dass die drei Abschirmwände im Wesentlichen eine U-Form einnehmen. Die Abschirmwände 22 sind jeweils aus einem Material ausgebildet, welches undurchlässig für die verwendete UV-Strahlung ist. Die Abschirmwände 22 schließen einen Bereich ein, in welchem die UV-Strahlung ausgesendet wird. Durch die Abschirmwände 22 kann die UV-Strahlung nicht in andere Bereiche der Reinigungseinheit 5 eindringen. Die US-Strahlung kann außerdem nicht aus dem Gehäuse 4' heraus. Dadurch ist die Reinigungseinheit 5' strahlungssicher ausgestaltet.

Auch wenn in den Figuren verschiedene Aspekte oder Merkmale der Erfindung jeweils in Kombination gezeigt sind, ist für den Fachmann - soweit nicht anders angegeben - ersichtlich, dass die dargestellten und diskutierten Kombinationen nicht die einzig möglichen sind. Insbesondere können einander entsprechende Einheiten oder Merkmalskomplexe aus unterschiedlichen Ausführungsbeispielen miteinander ausgetauscht werden.

### Bezugszeichenliste

- 1: Vorrichtung
- 2: Warenvorlaufband
- 3: Pfeil
- 4: Gehäuse
- 5: Reinigungseinheit
- 6: Tank
- 7: Einfüllöffnung
- 8: Reinigungstuch
- 9: Klettband
- 10: Magnet
- 11: Tropfsystem
- 12: Regal
- 13: Kassentisch
- 14: Pumpe
- 15: Sprühkopf
- 16: Bürste
- 17: Rollenbürste
- 18: Pfeil
- 19: Leuchteinheit
- 20: UV-Strahlung
- 21: Nebel

## Patentansprüche

1. Reinigungseinheit (5, 5') für ein Warenvorlaufband (2) eines Kassentisches (1),
wobei die Reinigungseinheit (5) Mittel zur UV-Bestrahlung des Warenvorlaufbandes (2) aufweist.

2. Reinigungseinheit (5, 5') für ein Warenvorlaufband (2) eines Kassentisches (1) nach Anspruch 1
**dadurch gekennzeichnet, dass**
die Reinigungseinheit (5) mit einer Leuchteinheit (19) ausgestattet ist, die UV-Strahlung aussendet.

3. Reinigungseinheit (5, 5') für ein Warenvorlaufband (2) eines Kassentisches (1) nach vorstehendem Anspruch
**dadurch gekennzeichnet, dass**
es sich bei der Leuchteinheit (19) um eine LED-Leuchteinheit handelt.

4. Reinigungseinheit (5, 5') für ein Warenvorlaufband (2) eines Kassentisches (1) nach einem der beiden vorstehenden Ansprüche **dadurch gekennzeichnet, dass**
die Leuchteinheit (19) innerhalb des Gehäuses (4') der Reinigungseinheit (5, 5') angeordnet und derart ausgerichtet ist, dass die UV-Strahlung (20) die Oberfläche des Warenvorlaufbandes (2) breitflächig trifft.

5. Reinigungseinheit (5, 5') für ein Warenvorlaufband (2) eines Kassentisches (1) nach einem der drei vorstehenden Ansprüche **dadurch gekennzeichnet, dass**
das Gehäuses (4') der Reinigungseinheit (5, 5') zwei zueinander parallel angeordnete Abschirmwände (22) aufweist und
dass die beiden Abschirmwände (22) durch eine weitere Abschirmwand (22) verbunden sind, so dass die drei Abschirmwände (22) im Wesentlichen eine U-Form einnehmen.

6. Reinigungseinheit (5, 5') für ein Warenvorlaufband (2) eines Kassentisches (1) nach vorstehendem Anspruch
**dadurch gekennzeichnet, dass**
die Abschirmwände (22) jeweils aus einem Material ausgebildet sind, welches undurchlässig für die verwendete UV-Strahlung ist.

7. Reinigungseinheit (5, 5') für ein Warenvorlaufband (2) eines Kassentisches (1) nach Anspruch 5
**dadurch gekennzeichnet, dass**
die Abschirmwände (22) einen Bereich einschließen, in welchem die UV-Strahlung ausgesendet wird, so dass die UV-Strahlung nicht in andere Bereiche der Reinigungseinheit (5) eindringen kann.

8. System aus einem Kassentisch und einer Reinigungseinheit nach einem der vorstehenden Ansprüche.
